# EUROPÄISCHE PATENTSCHRIFT

(11) **EP 2 446 268 B1**
(45) Veröffentlichungstag und Bekanntmachung des Hinweises auf die Patenterteilung: **01.05.2013**
(21) Anmeldenummer: 10726445.9
(22) Anmeldetag: 22.06.2010
(51) Int. Cl.: G01N 33/52, B01D 71/68

(54) **SPREITSCHICHT UND VERFAHREN ZUR HERSTELLUNG EINER ANALYSEELEMENT-SPREITSCHICHT**
SPREADING LAYER AND METHOD FOR PRODUCING AN ANALYSIS ELEMENT SPREADING LAYER
COUCHE D'ÉCARTEMENT ET PROCÉDÉ DE FABRICATION D'UNE COUCHE D'ÉCARTEMENT D'ÉLÉMENT D'ANALYSE

(30) Priorität: 24.06.2009 EP 09008233
(43) Veröffentlichungstag der Anmeldung: 02.05.2012
(73) Patentinhaber: Roche Diagnostics GmbH, 68305 Mannheim (DE); F.Hoffmann-La Roche AG, 4070 Basel (CH)
(72) Erfinder: HARTTIG, Herbert, 67434 Neustadt (DE); HILLER, Bernd, 68623 Lampertheim (DE); SCHWÖBEL, Wolfgang, 68309 Mannheim (DE); KASEJEW, Alexander, 68199 Mannheim (DE)
(74) Vertreter: Durm & Partner
(86) Internationale Anmeldenummer: PCT/EP2010/003751
(87) Internationale Veröffentlichungsnummer: WO 2010/149335

(56) Entgegenhaltungen:
- EP-A1- 1 640 716
- EP-A2- 1 114 997
- WO-A1-98/25758
- WO-A1-03/097219
- DE-A1- 2 133 848
- DE-A1-102007 035 583
- US-A- 6 110 369

## Beschreibung

Die vorliegende Erfindung betrifft ein Verfahren zur Herstellung einer Analyseelement-Spreitschicht, die dazu ausgebildet ist, in einem vorzugsweise optischen Analyseelement eine zu analysierende Flüssigkeit, z.B. eine Körperflüssigkeit aufzunehmen und über eine Flüssigkeitsaustrittsseite der Spreitschicht gleichmäßig zu verteilen. Die Erfindung betrifft auch eine Spreitschicht, die eine poröse Membran ist. Eine derartige Spreitschicht kann ein Teil eines Analyseelementes sein, das vor allem für medizinische Analysen verwendet wird. Ein wichtiges Anwendungsgebiet der Erfindung ist die Untersuchung sehr kleiner Probenvolumina, die typischerweise durch einen Einstich in die Haut eines Patienten gewonnen werden. Vorzugsweise sind die Körperflüssigkeitsprobenmengen kleiner als 1 µl.

Die Analyse erfolgt mittels eines Reagenzsystems, das bevorzugt aus mehreren Reagenzien und Hilfsstoffen besteht, die in das Analyseelement integriert sind. Die Reaktion der Reagenzien mit einem in der Körperflüssigkeit enthaltenen Analyten führt zu einer Messgröße, die für das gewünschte analytische Ergebnis charakteristisch ist und an dem Analyseelement gemessen werden kann. Speziell richtet sich die Erfindung auf so genannte optische Analysesysteme und Analyseelemente, bei denen die charakteristische Änderung des Analyseelementes, die ein Maß für die Menge und/oder das Vorhandensein eines Analyten ist, optisch messbar ist. In der Regel führt die Reaktion der Körperflüssigkeit in dem Analyseelement zu einer Änderung der Farbe in einer Analyseschicht, die Bestandteil des Analyseelementes ist und auch als Detektionsschicht bezeichnet wird. Die Farbänderung der Detektionsschicht wird photometrisch im Wellenlängenbereich von ca. 300 nm bis ca. 1400 nm vermessen. Neben colormetrischen Systemen sind auch andere optisch auswertbare Systeme bekannt, beispielsweise auf Fluoreszenzmessungen beruhende Systeme.

Zur qualitativen und quantitativen Bestimmung unterschiedlicher Analyten sind Analysesysteme in zahlreichen Varianten gebräuchlich. Besonders große Bedeutung haben Systeme zur Bestimmung der Glukosekonzentration im Blut von Diabetikern. Die Efindung ist insbesondere für solche Systeme geeignet, sie ist jedoch nicht darauf beschränkt. Derartige Systeme werden beispielsweise zur Überwachung des Gesundheitszustandes eines Patienten von ihm selbst durchgeführt ("home-monitoring"). Sie müssen deshalb einfach zu bedienen, möglichst klein und robust sein. Da für eine zuverlässige Beobachtung des Gesundheitszustandes täglich mehrere Messungen und somit auch mehrere Einstiche in die Haut durchgeführt werden müssen, soll die entnommene Blutmenge möglichst klein sein, so dass sie auch bei geringen Einstichtiefen und deshalb mit einen schmerzarmen Stich erfolgen kann.

Optische Analyseelemente haben in der Regel eine Tragstruktur, die aus Kunststoff besteht und oft ein länglicher Kunststoffstreifen ("Teststreifen") ist. An die Tragschicht des Analyseelementes grenzt eine so genannte Testschicht an, die mindestens einen Teil der Reagenzien und die Detektionsschicht umfasst. Die Testschicht kann aus einer oder mehreren Schichten bestehen, die in Fluidkontakt zueinander stehen und in der Regel parallel zueinander und zu der Tragschicht verlaufen. Reagenzienhaltige Schichten ("Reagenzschichten") der Testschicht bestehen oft aus einem saugfähigen porösen Schichtmaterial, wie beispielsweise Papier oder Vlies, in das die Reagenzien imprägniert sind.

Neben den saugfähigen porösen Materialien gibt es auch Testschichten, bei denen mindestens eine Reagenzschicht auf ein geeignetes transparentes Trägermaterial durch Beschichten aufgebracht wird. Zur Herstellung einer solchen, als Reagenzfilm bezeichneten CTL-Schicht (engl.: Coated Test Layer) werden in einem Verdickungsmittel gemischte Reagenzien in Form einer mehr oder weniger zähflüssigen Beschichtungsmasse als dünner Film auf das Trägermaterial aufgebracht und getrocknet. Bei Kontakt des Reagenzfilmes mit der wässrigen Probenflüssigkeit finden die für die Analyse erforderlichen Reaktionen mit den Reagenzien statt. Hierzu ist der Reagenzfilm in der Regel saugfähig und/oder quellfähig und/oder löslich. Derartige Systeme benötigen eine Flüssigkeitshaltestruktur, die mit dem Reagenzfilm in Fluidkontakt steht und eine Flüssigkeit gleichmäßig verteilt.

Die Präzision der analytischen Resultate ist bei optischen Analysesystemen und optischen Analyseelementen wesentlich von der Homogenität der Farbbildung in der Detektionsschicht der Testschicht abhängig. Um Inhomogenitäten zu reduzieren ist es gebräuchlich, eine zu der Testschicht des Analyseelementes benachbarte Spreitschicht vorzusehen, da eine gleichmäßige Verteilung der zu analysierenden Probenflüssigkeit über der Testschicht Voraussetzung für eine zuverlässige und aussagekräftige Analyse ist. Eine der Testschicht zugewandte Flüssigkeitsaustrittsseite der Spreitschicht steht mit einer Flüssigkeitseintrittsseite der Testschicht in Fluidkontakt. Die Spreitschicht ist so ausgebildet, dass eine in sie eindringende Flüssigkeit auf ihrer Flüssigkeitsaustrittsseite gleichmäßig verteilt wird. Bei der Bildung der Spreitschicht werden Materialen verwendet, in denen eine rasche Ausbreitung der Körperflüssigkeitsprobe über die gesamte Fläche der Schicht stattfindet. Die Ausbreitung geschieht in der Regel durch Kapillaraktivität der Spreitschicht. Geeignet sind deshalb lockere Faserverbundstrukturen, insbesondere Gewebe, Papiere oder Vliese aus hydrophilen oder hydrophilisierten Fäden und Fasern. Beispiele von derartigen Analyseelementen sind in DE 2118455 und DE 19629657 zu finden.

Um reproduzierbare Farbänderungen in der Nachweisschicht des Analyseelementes bei optischen Analysesystemen zu gewährleisten, ist eine gewisse Mindesthöhe einer Flüssigkeitsschicht mit dem Analyten notwendig, die an die Detektionsschicht des Analyseelementes angrenzt. Die Flüssigkeitsschichtdicke beträgt, insbesondere bei enzymatischen Tests, häufig zwischen 50 µm und 200 µm. Sie wird durch die entsprechend dimensionierten Spreitschichten sichergestellt.

Bei der Verarbeitung von kleinen Probenvolumina unterhalb von einem µl erweisen sich die bekannten Spreitstrukturen jedoch als nachteilig. Strukturen aus einem Feingewebe, die durch Fäden oder Fasern gewirkt oder gewebt sind, haben für sehr kleine Testschichten und Testschichtzonen, deren laterale Ausdehnung des Messflecks in Testschichtebene im Bereich von 100 µm bis 300 µm liegt, den Nachteil, dass die Maschenweite ebenfalls im Bereich von 100 µm liegt. Dies verursacht eine inhomogene Ausbildung der auf der Reaktion beruhenden Farbbildung in der Detektionsschicht, da unterhalb eines Fadens die Diffusion von Analyt aus der überstehenden Probe in die Chemieschicht behindert ist. Werden die Fäden zu einem Maschengewebe mit geringeren Maschenweiten verarbeitet, so entsteht ein im Vergleich zum Gesamtvolumen der Spreitstruktur zu geringes freies Volumen, in dem Flüssigkeit aufgenommen werden kann. Darüber hinaus ist die Dicke des Fadens zu groß im Vergleich zur Maschenweite, so dass eine optische Auswertung nicht mehr möglich ist. Die Verwendung dünnerer Fäden scheidet aus, weil damit keine ausreichende Schichthöhe in der Spreitschicht gewährleistet werden kann.

Auch Papiere oder Vliese eignen sich nicht für die Verarbeitung von kleinen Probenvolumina, da sie prinzipiell inhomogen sind und ein gewisse "Wolkigkeit" bei der optischen Vermessung aufweisen. Die hierdurch verursachten Inhomogenitäten in der Farbbildung sind bei großen Testschichtzonen und großen optischen Auswertebereichen durch Mittelwertbildung vernachlässigbar. Bei kleinen Testschichten mit einer Ausdehnung des Messflecks von unter 300µm in der Testschichtebene lassen sich jedoch keine zuverlässigen optischen Auswertungen vornehmen.

Der Erfindung liegt das technische Problem zugrunde, ein verbessertes Analyseelement und eine verbesserte Analyseelement-Spreitschicht herzustellen, die es insbesondere ermöglichen, mit extrem kleinen Probenmengen bei einem optischen Messverfahren eine sehr gute Messgenauigkeit zu gewährleisten. Darüber hinaus soll die Herstellung der Spreitschicht kostengünstig sein.

Gelöst wird das technische Problem durch eine Spreitschicht, die aus einer porösen Membran hergestellt ist. Erfindungsgemäß wird dazu ein Verfahren mit den Merkmalen des Anspruches 1 verwendet. Mittels eines Verfahrens mit den Merkmalen des Anspruchs 12 wird erfindungsgemäß ein optimiertes Analyseelement hergestellt. Eine hergestellte Spreitschicht und ein Analyseelement mit den Merkmalen des Anspruches 14 bzw. 15 lösen ebenfalls das zugrunde liegende technische Problem.

Im Rahmen der Erfindung wurde festgestellt, dass die Spreitschicht aus einer porösen Membran gewonnen werden kann. Allerdings erfüllen die bekannten, kommerziell verfügbaren Membranen nicht alle Anforderungen für eine Spreitschicht zum Nachweis von Analyten in einer Flüssigkeit, vorzugsweise in einer Körperflüssigkeit wie z.B. Blut. Um kurze Analysenzeiten zu ermöglichen müssen die Aufnahme und der Transport der Probenflüssigkeit schnell erfolgen. Das erfordert eine hydrophile Oberfläche der Porenstruktur, eine geeignete Porenstruktur und eine passend dimensionierte Gesamtdicke der Membran.

Poröse Polymermembranen sind in der Regel hydrophob. Durch Behandlung mit Netzmitteln und/oder Beschichtungen werden die äußeren und die Porenoberflächen der Membran hydrophil ausgerüstet und damit benetzbar für die Probe. Ein intrinsischer Kontaktwinkel von < 90 Grad reicht prinzipiell aus, bevorzugt sind jedoch Ausrüstungen mit einem intrinsischen Kontaktwinkel kleiner 70 Grad, besonders bevorzugt kleiner 60 Grad. Als intrinsischer Kontaktwinkel wird der Kontaktwinkel verstanden, der auf einer ideal glatten Oberfläche des betreffenden Materials beobachtet wird.

Über die Dicke betrachtet unterscheiden sich die Porenstrukturen verschiedener Membranen deutlich. Bei sogenannten symmetrischen Membranen ist die Porengröße auf beiden Außenseiten einer Membran gleich. Bei den sogenannten asymmetrischen Membranen ist die Porengröße auf beiden Seiten unterschiedlich. Besonders bei hoch asymmetrischen Membranen unterscheiden sich die Porengrößen um mehr als eine Größenordnung.

Die Oberflächen einer Membran weisen in der Regel eine andere Verteilung der Porengrößen auf, als die unmittelbar darunter liegende Schicht. Dies trifft besonders auf Phaseninversionsmembranen zu. Dort weist häufig die Membranseite, die beim Herstellprozess dem Fällmittel zugewandt war, eine hautartige Oberfläche auf. Eine solche Oberfläche ist dadurch gekennzeichnet, dass die Fläche, die von offenen Poren gebildet wird, im Vergleich zur Fläche, die durch das Membranmaterial gebildet wird, kleiner ist, als bei entsprechenden Flächen im Inneren der Membran. Der Anteil der Fläche, die von offenen Poren gebildet wird, an der gesamten geometrischen Fläche einer Oberfläche wird im Folgenden als "freie Porenfläche" bezeichnet. Die Einheit dieser relativen Öffnungsfläche ist Prozent.

Aufgrund der zunehmenden Kapillaraktivität von kleineren Poren wird Flüssig, keit bevorzugt von kleinen Poren aufgenommen. Ein kapillarer Transport erfolgt bevorzugt von großen zu kleinen Poren. Wird also eine Probe auf die großporige Seite einer Membran aufgegeben, so erfolgt, ausreichende Hydrophilie vorausgesetzt, eine schnelle Aufnahme der Probe und ein Flüssigkeitstransport zur feinporigen Seite. Auf der feinporigen Seite erfolgt bevorzugt ein Ausspreiten der Probe in den kleinen Poren. Die großen Poren werden nur sehr langsam gefüllt, ausreichendes Probenvolumen vorausgesetzt. Mit zunehmender Feinheit der Poren nimmt auch der Strömungswiderstand gemäß dem Hagen-Poiseuille-Gesetz zu, so dass die Transportgeschwindigkeit bei sehr kleinen Poren trotz hoher Kapillaraktivität niedrig ist. Auch über lange Strecken, im Bereich von mehreren Millimetern, ist der Transport durch kleine Poren langsam. Wird eine Probe auf die feinporige Seite einer Membran aufgegeben, so erfolgt bevorzugt ein Ausspreiten der Probe.

Bei symmetrischen Membranen erfolgen Spreitung und Eindringen einer aufgebrachten Flüssigkeitsprobe in die Membran gleichermaßen. Ein bevorzugter Transport findet nicht statt.

Reale Membranen sind in der Regel weder exakt symmetrisch noch gleichförmig asymmetrisch. Für jede Membran ist eine genaue Analyse der Struktur und des Transportverhaltens notwendig. So wird beispielsweise bei Membranen vom Typ MMM der Fa. Pall, Dreieich Deutschland beobachtet, dass die größten Poren sich in etwa in der Mitte der Membrandicke befinden. Das hat zur Folge. dass eine auf die Oberfläche der Membran aufgebrachte Flüssigkeitsprobe bevorzugt ausspreitet und nur sehr langsam die Membrandicke durchdringt. Bei kleinen Probevolumina erreicht die Probe nicht die gegenüberliegende Seite. Solche Membranstrukturen sind für Spreitschichten nicht geeignet

Kommerziell verfügbare Membranen die einen schnellen Durchlass einer Flüssigkeit und eine hinreichend schnelle Verteilung ermöglichen, sind asymmetrisch und weisen auf der feinporigen Seite Poren mit einer mittleren Porengröße auf, die kleiner 1 µm ist Asymmetrische Membranen sind beispleiweise aus den Druckschriften WO 03/097219 WO 98/25758, US 6.110.369. EP 1 640 716 und EP 1 114 997 bekannt.

Steht eine solche Membran in engem hydraulischem Kontakt mit einer porösen, saugenden Reagenzschicht, so wurde erkannt, dass bei Blut als Probeflüssigkeit Hämoiyse auftritt. Die Ursache wird darin vermutet, dass Erythrozyten in Poren kleiner 3 µm bis 4 µm festgehalten werden und die Kapillaraktivität der saugenden Nachweisschicht einen ausreichenden Differenzdruck erzeugt, um die Erythrozyten zum Platzen zu bringen. Membranen die Erythrozyten über ihre Dicke so abtrennen, dass keine Hämolyse auftritt, müssen jedoch eine sehr große Dicke von ca. 300 µm haben und erfordem ein zu großes Blutvolumen. Ihre Verwendung als Spreitschicht scheidet deshalb auch aus.

Im Rahmen der Erfindung wird unter dem Begriff "mittlere Porengröße einer Schicht" der Mittelwert der Porengröße der Poren in der Schicht verstanden. Da sich diese mittlere Porengröße in der Regel nicht mehr durch Vermessung der einzelnen Poren bestimmen lässt, wird die mittlere Porengröße so definiert, dass die Schicht Partikel mit der der mittleren Porengröße entsprechenden Größe zu 85 Prozent zurückhält. Ist die mittlere Porengröße beispielsweise 1 µm, so werden durch die Schicht 85 Prozent aller Partikel mit einer Größe von mindestens 1 µm nicht durchgelassen.

Andere bekannte Membranen, beispielsweise solche, die durch Schälverfahren hergestellt werden, wie etwa in DE 2133848 oder in DE 10 2007 035 583 beschrieben, weisen eine niedrige Permeabilität und eine vergleichsweise hohe Dicke auf, die sie für den Einsatz als Spreitschicht zur Blutanalyse ungeeignet machen. Darüber hinaus ermöglichen sie keine gezielte Transportrichtung einer Flüssigkeitsprobe.

Im Rahmen der Erfindung wurde erkannt, dass asymmetrisch ausgebildete Membranen generell als Spreitschicht geeignet sein könnten, da sie eine gezielte Transportrichtung einer Probe gewährleisten. Jedoch weisen die bekannten asymmetrischen Membranen eine nicht geeignete Porengröße auf. Die Membranen sind derart ausgebildet, dass die mittlere Porengröße an ihrer einen Seite wenigstens doppelt so groß ist wie die mittlere Porengröße auf der gegenüberliegenden Seite der Membran. Bevorzugt ist das Verhältnis der mittleren Porengröße von einer Seite der Membran zu der gegenüberliegenden Seite auch größer, beispielsweise 3 zu 1, 5 zu 1, 10 zu 1 bis hin zu 100 zu 1 oder 200 zu 1.

Im Rahmen der Erfindung wurde erkannt, dass eine poröse Membran, deren mittlere Porengrößen über deren Dicke wenigstens abschnittsweise zunimmt, zur Herstellung einer Spreitschicht geeignet ist. Bevorzugt wird eine Ausgangsmembran verwendet, die eine asymmetrische Membran ist, bei der die mittlere Porengröße von einer Seite der Membran zu der gegenüberliegenden Seite zunimmt. Besonders bevorzugt ist eine Membran, bei der die mittlere Porengröße über die Membrandicke stetig zunimmt. Das erfindungsgemäße Verfahren zur Herstellung einer Analyseelement-Spreitschicht umfasst die folgenden Schritte:
Eine poröse Ausgangsmembran mit einer Unterseite und einer Oberseite und einer Ausgangsdicke D zwischen der Unterseite und der Oberseite wird bevorzugt einer Vorrichtung zum Entfernen einer Teilschicht zugeführt. Die Ausgangsmembran ist so ausgebildet, dass die mittlere Größe der Poren über deren Ausgangsdicke wenigstens abschnittsweise zunimmt. Als Oberseite wird im Weiteren die Seite bezeichnet, welche die größeren Poren aufweist und auf welche bei der Verwendung der Membran als Spreitschicht die Probenflüssigkeiten aufgegeben werden. In einem weiteren Verfahrensschritt wird eine Teilschicht der Ausgangsmembran in einem vorgegebenen Abstand zu deren Oberseite dadurch entfernt, dass die Ausgangsmembran parallel zur Oberseite in dem entsprechenden Abstand geschnitten wird. Nicht nur das Schneiden bzw. Durchtrennen mit einem Messer oder einer Klinge wird als "Schneiden" bezeichnet, sondern auch das Schneiden mit einem schnell bewegten Werkzeug, z. B. mit einer Fräse oder Rotationsfräse. Alternativ könnte die Ausgangsmembran auch mittels eines Laserstrahls oder eines Wasserstrahls oder Ähnlichem geschnitten werden.

Die nach dem Schneiden der ersten Teilschicht verbleibende zweite Teilschicht der Ausgangsmembran ist die Spreitschicht. Ihre Dicke d ist geringer als Ausgangsdicke D der Ausgangsmembran. Die Poren der Spreitschicht stehen derart miteinander in Fluidverbindung, dass sie für eine Flüssigkeit so durchlässig sind, dass eine Verteilung der Flüssigkeit an der Flüssigkeitsaustrittsseite der Spreitschicht erfolgt. Dabei findet selbstverständlich auch ein Flüssigkeitstransport von der Flüssigkeitseintrittsseite zu der Flüssigkeitsaustrittsseite statt.

Erfindungsgemäß kann die so hergestellte Spreitschicht zur Herstellung eines Analyseelements zur Analyse einer Flüssigkeitsprobe dienen, die gleichmäßig auf dem Analyseelement verteilt wird. Das Analyseelement hat ein Testfeld und die oben beschriebene Analyseelement-Spreitschicht. Das Testfeld (Basiselement, Testelement) umfasst eine Tragschicht und eine Testschicht, die wenigstens eine Reaktionsschicht (Nachweisschicht) mit wenigstens einem Reagenz aufweist, welches Teil eines Reagenzsystems zum (optischen) Nachweis eines Analyten in der Flüssigkeitsprobe ist. Der Aufbau eines derartigen unvollständigen Testfelds, das auch Teststreifen genannt wird, ist im Stand der Technik hinlänglich bekannt. Insbesondere sind solche Testfelder (Basiselemente) zur Herstellung des Analyseelements geeignet, bei denen die Testschicht in Form einer dünnen CTL-Schicht auf die Tragschicht beschichtet ist. Diesen Testfeldern fehlt eine Flüssigkeitshaltestruktur, die bei dem erfindungsgemäßen Analyseelement durch die hergestellte Spreitschicht gebildet wird.

Nachdem die Spreitschicht dem "unvollständigen" Testelement (Testfeld) zugeführt wurde, wird die Spreitschicht auf das noch feuchte Testfeld derart aufgebracht, dass die Flüssigkeitsaustrittsseite der Spreitschicht an der Oberseite der Testschicht anliegt. Die Oberseite der Testschicht ist die Seite, die der Tragschicht gegenüberliegt. Bevorzugt wird die Spreitschicht dabei derart gebogen, dass sie auf das Testelement "aufgerollt" wird. Durch das Aufbringen werden die Spreitschicht und das Testfeld miteinander verbunden, so dass sie gemeinsam das erfindungsgemäße Analyseelement bilden.

Bevorzugt wird im Zeitpunkt des Zusammenführens, wenigstens jedoch im Zeitpunkt des Aufbringens der Spreitschicht, ein Feuchtigkeitsfilm auf der Oberseite des Testfelds gebildet, der eine Dicke von wenigstens einigen µm hat. Durch diesen Feuchtigkeitsfilm wird die Spreitschicht an dem Testfeld gehalten. Die Spreitschicht ist eine asymmetrische Membran, die bereits getrocknet ist und die bevorzugt als hydrophile Membran vorliegt oder hydrophilisiert ist.

Wichtig bei diesem "Auflaminieren" der Spreitschicht auf das Testfeld ist es, dass die Testschicht hinreichend feucht ist. Bei einem mehrschichtigen Aufbau des Testfelds muss die letzte, also oberste Schicht, feucht sein, so dass die (der Tragschicht gegenüberliegende) Oberseite des Testfelds noch feucht ist. Als feucht im Sinne der Erfindung wird eine relative Feuchte von wenigstens 25 % und höchstens 50 % verstanden. Relative Feuchte bezeichnet den Gewichtsanteil von Wasser in der Testschicht.

Nach dem Aufbringen der Spreitschicht auf das Testfeld wird das entstandene Analyseelement getrocknet und ein insgesamt einstückiges Analyseelement gebildet. Überraschenderweise weist das Analyseelement mit der auflaminierten Membran nach dem Trocknen gegenüber den bekannten Analyseelementen mit einem herkömmlichen Spreitnetz verbesserte Eigenschaften auf. Insbesondere zeigt sich eine geringere Streuung der Messergebnisse. Bei der Untersuchung von Vollblut wird ein deutlich geringerer Einfluss des Hämatokrit auf die Testergebnisse verzeichnet.

Im Rahmen der Erfindung wurde erkannt, dass entgegen den Erwartungen von Fachleuten die Eignung derartiger Analyseelemente für Analysen von Flüssigkeitsproben, insbesondere von Vollblut, gegeben ist. Es wurde befürchtet, dass die Kinetik der Reaktion durch die auflaminierte Membran beeinflusst wird. Speziell bestanden Bedenken in der Fachwelt, dass Enzyme oder Indikatoren aus der Testschicht, insbesondere dem Reagenzsystem, während der Herstellung in die Membran (Spreitschicht) eingesaugt werden könnten und auf diese Weise das Analyseelement für die Analyse unbrauchbar machen würden. Es wurde weiter befürchtet, dass während der Analyse einer Flüssigkeit die in die Membran eingesaugten Enzyme und Indikatoren zu einer Reaktion führen, die der optischen Auswertung und Kontrolle nicht zugänglich sind. Erwartet wurde, dass der Signalhub bei der Messung deutlich verflachen würde und damit die Auswertung erschwert würde. All diese Bedenken der Fachwelt haben sich jedoch als unbegründet erwiesen. Vielmehr zeichnet sich das erfindungsgemäße Analyseelement durch verbesserte Messeigenschaften aus, insbesondere durch geringere Streuung der Ergebnisse und ist insbesondere für die Untersuchung von kleinen Probevolumina besonders geeignet.

Die durch das Entfernen der ersten Teilschicht der Ausgangsmembran neu gebildete Membranoberfläche ist die Flüssigkeitsaustrittsseite der Spreitschicht. Die mittlere Porengröße an der Flüssigkeitsaustrittsseite der Spreitschicht beträgt mindestens 1 µm. Bevorzugt ist die mittlere Porengröße an der Flüssigkeitsaustrittsseite mindestens 2 µm oder mindestens 3 µm, besonders bevorzugt mindestens 5 µm groß. Eine derartige Porengröße gewährleistet, dass keine Hämolyse bei der Verwendung von Blut als Körperflüssigkeitsprobe auftritt.

Die Flüssigkeitsaustrittsseite der Spreitschicht zeichnet sich dadurch aus, dass sie eine freie Porenfläche aufweist, die mindestens 70 %, bevorzugt mindestens 80 % und besonders bevorzugt mindestens 90 % beträgt. Somit unterscheidet sich die erfindungsgemäße Spreitschicht deutlich von den bisher bekannten Spreitschichten.

In einer bevorzugten Ausführungsform wird eine Teilschicht der Ausgangsmembran nur an ihrer Unterseite entfernt, so dass die Oberseite der Ausgangsmembran die Oberseite der Spreitschicht bildet, die die Flüssigkeitseintrittsseite ist. Die der Oberseite gegenüberliegende Seite bildet die neu entstandene Membranoberfläche und ist die Flüssigkeitsaustrittsseite der Spreitschicht.

Vorzugsweise wird eine Ausgangsmembran zur Durchführung des erfindungsgemäßen Verfahrens verwendet, deren mittlere Porengröße an der Oberseite mindestens 10 µm, bevorzugt mindestens 20 µm und bevorzugt mindestens 30 µm beträgt. Im Rahmen der Erfindung wurde festgestellt, dass in einer bevorzugten Ausführungsform die verwendete Ausgangsmembran eine mittlere Porengröße an der Oberseite hat, die höchstens 60 µm, bevorzugt höchstens 50 µm, besonders bevorzugt höchstens 40 µm beträgt. Eine derartige Ausgangsmembran ist besonders geeignet, da die Oberseite der Ausgangsmembran gleichzeitig die Oberseite der Spreitschicht bildet und somit die Poren an der Flüssigkeitseintrittsseite der Spreitschicht entsprechend groß sind.

Eine derartige mittlere Porengröße an der Oberseite, die die Flüssigkeitseintrittsseite der Spreitschicht bildet, hat den Vorteil, dass bei Kontakt der Spreitschicht mit einem Stechelement mit Kapillarkanal, von dem die Probenkörperflüssigkeit bei einem Stich in die Haut aufgenommen wird, durch Kapillarkräfte die Probenflüssigkeit mit extrem hoher Zuverlässigkeit aus dem Kapillarkanal des Stechelementes in die Spreitschicht eingesaugt wird. Die Kapillarwirkung der im Vergleich zum Kapillarkanal deutlich geringeren Porengröße sorgt dafür, dass die Spreitschicht die Flüssigkeit aufnimmt.

Durch die über die Dicke der Spreitschicht zur Flüssigkeitsaustrittsseite geringer werdende Porengröße wird die Kapillarwirkung erhöht. Somit wird sichergestellt, dass die in die Spreitschicht eintretende Flüssigkeit zur Flüssigkeitsaustrittsseite übertragen wird, an die die Testschicht (Testfeld) des Analyseelementes angrenzt.

Bevorzugt ist deshalb die mittlere Porengröße an der Flüssigkeitsaustrittsseite der Spreitschicht höchstens 20 µm, bevorzugt höchstens 10 µm, besonders bevorzugt höchstens 7 µm.

Im Rahmen der Erfindung wurde festgestellt, dass eine stark asymmetrische Membran als Ausgangsmembran bevorzugt ist. Die stark asymmetrische Membran zeichnet sich dadurch aus, dass die mittlere Porengröße über ihre Dicke, das heißt über ihren Querschnitt stark zunimmt. Typischerweise ist das Verhältnis von der mittleren Porengröße der Oberseite zu der mittleren Porengröße der Unterseite etwa 1 : 40 bis 1 : 200, teilweise bis 1 : 1000.

Von der Ausgangsmembran ist die erste Teilschicht derart abzuschneiden, dass an der gebildeten Spreitschicht die Flüssigkeitsaustrittsseite eine Porengröße von höchstens 20 µm, bevorzugt höchstens 10 µm, besonders bevorzugt höchstens 7 µm beträgt. Auf diese Weise sind die "kleinsten Poren" (kleinste mittlere Porengröße) an der Flüssigkeitsaustrittsseite der Spreitschicht so groß, dass - unter den Anwendungsbedingungen des Analyseelementes und der optischen Analyse von Blut - keine Hämolyse verursacht wird. Durch das Abtrennen der "feinporigen" Teilschicht entsteht eine für ein optisches Analyseelement zur Blutanalyse geeignete Spreitschicht aus einer an sich ungeeigneten asymmetrischen Membran. Weiterhin anwendbar ist die erfindungsgemäße Spreitschicht auch in Analysesystemen und Analyseelementen, die elektrochemisch die Menge und/oder das Vorhandensein eines Analyten messen.

Für das Schneiden eignet sich insbesondere eine Ausgangsmembran, die aus Polyethersulfon (PESu) besteht und mit Hydroxypropylcellulose hydrophilisiert ist. Als Ausgangsmembran ist beispielsweise eine Membran des Typs BTS 25 der Fa. Pall, Dreieich, Deutschland, geeignet. Eine derartige Membran hat einen "Bubblepoint" von 25 psi und eine kleinste mittlere Porengröße von nominell 0,45 µm. Dieser Nominalwert wird durch Filtrationsexperimente ermittelt. Dabei erhält die Membran Partikel mit einem Durchmesser von 0,45 µm zu 85 % zurück, das heißt, 85 % aller Partikel dieser Größe werden von der Membran nicht durchgelassen.

Eine ca. 126 µm dicke asymmetrische Ausgangsmembran hat an der Oberseite die erwähnte mittlere Porengröße von 0,45 µm und an der Unterseite eine Porengröße von 45 µm. Nach dem Schneiden entsteht eine Spreitschicht mit einer Dicke von 82 µm und einer Porengröße der durch das Schneiden gebildeten Membranoberfläche von 5,5 µm im Mittel. Eine derartige Membran, bei der die kleinste mittlere Porengröße 5,5 µm beträgt, ist für die Verwendung als Spreitschicht zur optischen Blutanalyse sehr gut geeignet.

In einem alternativen Verfahren kann eine Ausgangsmembran des Typs BTS 65 der Fa. Pall, Dreieich, Deutschland, eingesetzt werden. Eine derartige Membran hat einen "Bubblepoint" von 63 psi und eine kleinste mittlere Porengröße von nominell 0,1 µm. Andere Membranen aus der gleichen Produktfamilie, wie z.B. BTS 55, BTS 45, BTS 25 können in vergleichbarer Weise verwendet werden. Die Ausgangsmembran wird mit einer Flüssigkeit wie Wasser oder einem geschmolzenem hydrophilen Wachs, wie z.B. PEG 1500 getränkt, mit der Oberseite, der großporigen Seite, auf eine Unterlage aufgelegt und so weit abgekühlt, dass die eingetränkte Flüssigkeit erstarrt. Dabei bildet die erstarrte Flüssigkeit eine kraftschlüssige Verbindung zwischen der Membran und der Unterlage. Die Unterlage wird mit einem Abstand, der der gewünschten Dicke der Spreitschicht entspricht, unter einem Schneidwerkzeug, z.B. einem horizontal angeordnetem Walzenfräser bewegt. Der Walzenfräser entfernt die feinporige Teilschicht. Durch Zuführung von Wärme wird die Flüssigkeit geschmolzen und im Falle von PEG mit Wasser ausgespült. Nach dem Trocknen steht eine Membran, die als Spreitschicht verwendet werden kann, zur Verfügung. Bevorzugt ist die Membran hydrophil oder sie ist hydrophilisiert.

Für eine kontinuierliche Fertigung wird eine Ausgangsmembran als Bahnware eingesetzt. Als Unterlage dient z.B. eine Rolle oder Walze, die über ihren Umfang mindestens zwei Temperaturzonen aufweist. Im Zulaufbereich der Membran bis zur Position des Schneidwerkzeugs ist die Unterlage auf einem Temperaturniveau unterhalb des Schmelzpunktes der Tränkflüssigkeit. Im nachfolgenden Bereich ist die Unterlage auf einem Temperaturniveau oberhalb des Schmelzpunktes der Tränkflüssigkeit. Als Schneidwerkzeug dient ein feststehendes Messer, ein horizontal vibrierendes Messer, ein Walzenfräser oder ein anderes geeignetes spanabhebendes Werkzeug. Die Membran wird vor dem Zulauf auf die Walze mit der Tränkflüssigkeit getränkt, gegebenenfalls bei erhöhter Temperatur. Überschüssige Tränkflüssigkeit wird mit im Stand der Technik bekannten Mitteln abgestreift. Die getränkte Membran wird auf die (rotierende) Walze aufgerollt und friert an. Die unerwünschte Teilschicht wird entfernt. Im Fortgang der Rotation der Walze wird Wärmeenergie z.B. durch Wärmestrahlung, Warmluft oder Warmwasser zugeführt und die Tränkflüssigkeit schmilzt. Die verbliebene Membran wird von der Walze abgezogen, in einer nächsten Station ggf. mit Wasser gewaschen und von der Tränkflüssigkeit frei gespült. In einer weiteren Station erfolgt dann die Entfernung des Wassers durch Trocknung und das Produkt, die Spreitschicht, kann auf eine Rolle aufgewickelt oder weiterverarbeitet werden.

Beispielsweise kann die Spreitschicht zur Herstellung eines optischen Analyseelements verwendet werden. Die trockene Spreitschicht wird dann einem Testfeld mit einer transparenten Tragschicht und einer Testschicht zugeführt und auf die Oberseite der Testschicht, die der Tragschicht abgewandt ist, auflaminiert. Die Spreitschicht und das Testfeld sind durch das Laminieren dauerhaft miteinander verbunden und bilden gemeinsam das Analyseelement.

Die Erfindung wird nachfolgend anhand von in den Figuren dargestellten besonderen Ausführungsformen näher erläutert. Die dort dargestellten Besonderheiten können einzeln oder in Kombination verwendet werden, um bevorzugte Ausgestaltungen der Erfindung zu schaffen. Zur Verdeutlichung von Details stimmen die dargestellten Proportionen der einzelnen Komponenten teilweise nicht. Die beschriebenen Ausführungen stellen keine Einschränkung der durch die Ansprüche in ihrer Allgemeinheit definierten Erfindung dar. Es zeigen:
- Figur 1: ein Prinzipbild einer Schneidvorrichtung zum Abtragen einer Teilschicht einer Ausgangsmembran:
- Figur 2: ein Prinzipbild zum Entfernen einer Teilschicht der Ausgangsmembran mittels einer Schneidvorrichtung;
- Figur 3: ein Prinzipbild einer alternativen Schneidvorrichtung;
- Figur 4: eine aus einer Ausgangsmembran hergestellte Spreitschicht;
- Figur 5a, b: eine Membranoberfläche einer Membran;
- Figur 6: ein Prinzipbild der Herstellung eines Analyseelements
- Figur 7: ein Prinzipbild einer Herstellungsanlage zur Herstellung eines Analyseelements;
- Figur 8a, b: den zeitlichen Verlauf von Vollblutmessungen mit unterschiedlichem Glucosegehalt an zwei Analyseelementen;
- Figur 9a, b: den zeitlichen Verlauf von Vollblutmessungen mit unterschiedlichen Hämatokritwerten an zwei Analyseelementen.

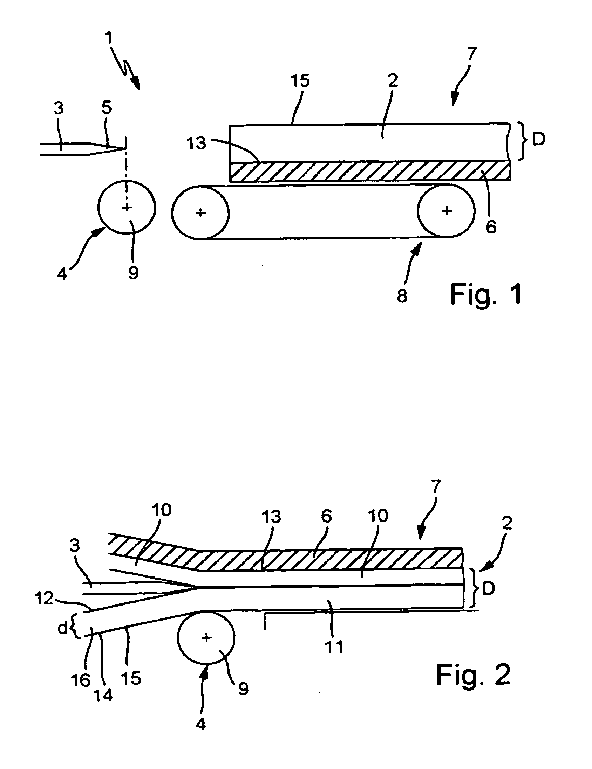

Fig. 1 zeigt eine Schneidvorrichtung 1, mit der eine Ausgangsmembran 2 derart in zwei Teilschichten geteilt werden kann, dass eine erfindungsgemäße Spreitschicht hergestellt wird. Die Schneidvorrichtung 1 schließt ein Schneidmesser 3 mit einer scharfen Klinge 5 ein. Das Schneidmesser 3 kann beispielsweise nach Art einer Rasierklinge oder ähnlich einem Skalpell ausgebildet sein.

Bevorzugt umfasst die Schneidvorrichtung 1 auch eine Ablenkvorrichtung 4, durch die die Ausgangsmembran 2 während des Produktionsprozesses umgelenkt wird. Die Ausgangsmembran 2 wird beim Passieren der Ablenkvorrichtung 4 durch die Schneidvorrichtung 1 geschnitten. In einer bevorzugten Ausführungsform ist die Ablenkvorrichtung 4 als Walze 9 ausgebildet, wie in Fig. 1 gezeigt, die bevorzugt rotierbar ist.

Um die Ausgangsmembran 2 dem Schneidmesser 3 zuzuführen, kann beispielsweise ein Förderband 8 verwendet werden, das derart angeordnet ist, dass die Oberseite des Förderbands 8 tangential zur Walze 9 verläuft. In einer bevorzugten Ausführungsform kann die Ablenkvorrichtung 4 alternativ zu der rotierbaren Walze 9 eine feststehende Kante aufweisen, an der die Ausgangsmembran 2 abgelenkt wird. Bevorzugt ist die feststehende Kante abgerundet. Die Kante erfüllt dabei die Funktion der Walze 9 und ist so angeordnet, dass die Ausgangsmembran beim Passieren der Kante geschnitten wird.

Fig. 1 ist zu entnehmen, dass die Ausgangsmembran 2 auf einer Unterlage 6 befestigt ist und gemeinsam mit der Unterlage 6 eine Materialbahn 7 bildet, die zu der Schneidvorrichtung 1 bewegt wird. Die als Träger dienende Unterlage 6 kann beispielsweise ein Klebstreifen oder ein Klebeband sein. Möglich ist auch, eine Klebeschicht zwischen der Unterlage 6 und der Ausgangsmembran 2 vorzusehen. Bevorzugt wird die Ausgangsmembran 2 mit ihrer Unterseite 13 auf die Unterlage 6 aufgeklebt. Die Materialbahn 7 ist derart auf dem Förderband 8 angeordnet, dass die Unterlage 6 auf dem Förderband 8 liegt und beim Passieren der Walze 9 mit dieser in Kontakt kommt. Beim Abtrennen einer Teilschicht von der bevorzugt mindestens 80 µm, besonders bevorzugt mindestens 120 µm dicken Ausgangsmembran 2 wird die obere Teilschicht von der unteren Teilschicht mit der Unterlage 6 entfernt. Die obere Teilschicht ist dann die gewünschte Spreitschicht, deren Dicke d mindestens 30 µm, bevorzugt mindestens 50 µm und besonders bevorzugt mindestens 70 µm beträgt.

Fig. 2 zeigt eine Detailzeichnung der Schneidvorrichtung 1 aus Fig. 1, jedoch ohne Förderband. An Stelle des Förderbands ist eine ebene Platte vorgesehen, über die die Materialbahn 7 mit der porösen Ausgangsmembran 2 manuell der Schneidvorrichtung 1 zugeführt wird.

In einer bevorzugten Ausführungsform des Herstellungsverfahrens wird die Ausgangsmembran 2 mit der Oberseite 15 über die Ablenkvorrichtung 4 bewegt. Bevorzugt ist die Ausgangsmembran 2 mit ihrer feinporigen Oberseite 13 auf der Unterlage 6 befestigt. Beim Zuführen der Ausgangsmembran 2 zum Schneidmesser 5 ist die Unterlage 6 nach oben orientiert, während die Unterseite 15 mit der Walze 9 in Kontakt kommt. Durch eine derartige Anordnung der Ausgangsmembran 2 können Dickenvariationen des Klebebandes bzw. der Klebeschicht zwischen Ausgangsmembran 2 und Unterlage 6 eliminiert werden.

Bevorzugt ist die Position des Schneidmessers 3 und dessen Klinge 5 (bevorzugt parallel) zur Ablenkvorrichtung 4 fixiert. Insbesondere ist der Abstand zwischen der Klinge 5 und der Oberfläche der Walze 9 konstant. Durch die Kopplung der Klinge 5 des Schneidmessers 3 mit der Walze 9 wird eine Exzentrizität der Walze kompensiert. Da die Oberseite 15 der Membran 2 und somit die Teilschicht 11 der Membran 2, die nach dem Schneiden die Spreitschicht 16 bilden wird, über die Walze 9 bewegt wird, führt die Kopplung zu einer sehr hohen Präzision der Dicke der herzustellenden Spreitschicht 16.

Die Klinge 5 ist quer zur Bewegungsrichtung der Ausgangsmembran 2 und parallel zur Rotationsachse der Walze 9 bewegbar. Sie schwingt in der Bewegungsebene der Ausgangsmembran 2. Bevorzugt ist eine schnelle Schwingung der Klinge 5, beispielsweise mit Ultraschall. Diese Querschwingungen sind derart, dass der Abstand der Klinge 5 zur Oberfläche der Walze 9 stets konstant bleibt. Durch die bewegte Klinge 5 wird ein sehr exakter Schnitt ermöglicht.

Im Rahmen der Erfindung wurde festgestellt, dass die Schneidqualität und die Güte der erzeugten Membranoberfläche der Spreitschicht durch ein Befeuchten der Ausgangsmembran mit Wasser oder eines anderen als Schmiermittel wirkenden Stoffes deutlich verbessert werden kann. Der Schneidwiderstand der befeuchteten Membran ist erheblich reduziert. Das Befeuchten der porösen Ausgangsmembran kann vor oder während des Entfernens der einen Teilschicht der Ausgangsmembran 2 erfolgen. Wenigstens während des Abtrennens sollte die Membran feucht sein.

Alternativ kann die Porenstruktur der Ausgangsmembran 2 auch mit Flüssigkeiten gefüllt werden, die vor dem Abtrennen der Teilschicht auf eine Temperatur unterhalb des Schmelzpunktes der Flüssigkeit gebracht werden. Neben Wasser sind Wachse geeignet, bevorzugt wasserlösliche Wachse aus der Familie der Polyethylenoxide. Sie werden nach dem Schneiden und Abtrennen der Spreitschicht wieder aus den Poren gelöst. Dies kann beispielsweise in einem Waschprozess mit entsprechenden Temperaturen erfolgen. Anschließend kann die Spreitschicht getrocknet und aufgewickelt werden. Alternativ ist es selbstverständlich auch möglich, die Spreitschicht direkt nach dem Trocknen auf eine Testschicht eines Analyseelements zu befestigen, beispielsweise zu laminieren. Möglich ist es folglich, ein mit einer Testschicht beschichtetes Trägermaterial mit der Spreitschicht durch Laminieren zusammenzufügen, um ein Analyseelement zu bilden.

Während des Schneidvorgangs von Fig. 2 wird die mit der Unterseite 13 an der Unterlage 6 aufgeklebte Ausgangsmembran 2 derart geschnitten, dass eine erste Teilschicht 10 mit den feinporigen Poren, deren Porengröße für die Funktion einer Spreitschicht zu gering ist, abgetrennt und über die Klinge 5 hinweg bewegt wird. Die zweite Teilschicht 11 wird zwischen der Klinge 5 und der Walze 9 entlang geführt. Sie hat die präzise, gewünschte Dicke d von bevorzugt ca. 40 µm bis ca. 100 µm und bildet die Spreitschicht 16. Die neu gebildete Membranoberfläche der Spreitschicht 16 ist die Flüssigkeitsaustrittsseite 12 der Spreitschicht 16. Die Oberseite 15 der Ausgangsmembran 2 bildet die Oberseite der Spreitschicht 16, die die Flüssigkeitseintrittsseite 14 ist.

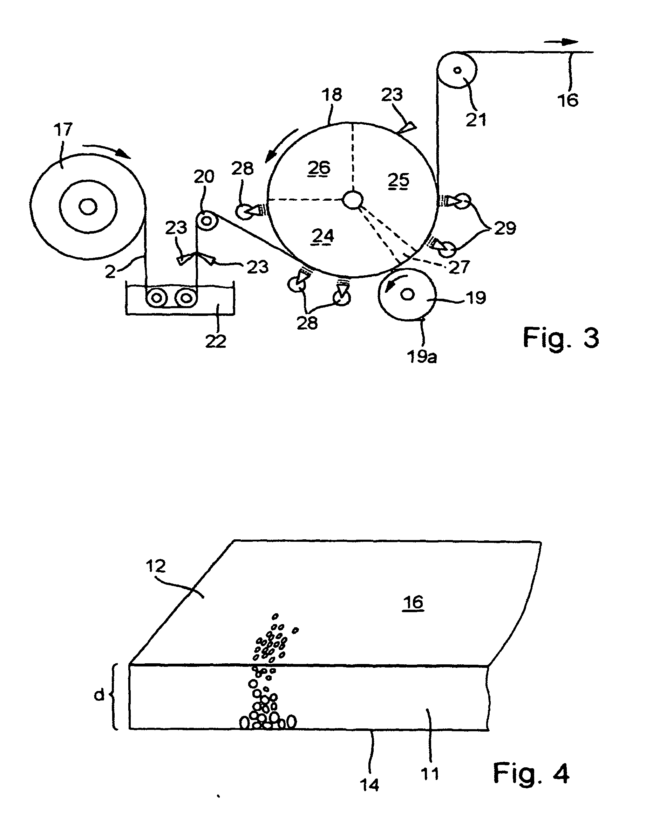

Eine alternative Schneidvorrichtung 1 ist in Figur 3 gezeigt. In dieser Ausführungsform liegt die Ausgangsmembran 2 als Bahnware vor und wird von einer Ausgangsmembranrolle 17 abgewickelt. Die Ausgangsmembran 2 wird durch eine Tränkstation 22 geführt, in der sie mit einer Tränkflüssigkeit, beispielsweise Wasser oder geschmolzenem Wachs, getränkt wird.

An einem Abstreifer 23, der in Bewegungsrichtung hinter der Tränkstation 22 angeordnet ist, wird überschüssige Tränkflüssigkeit abgestreift. Die Ausgangsmembranbahnware wird an einer ersten Umlenkrolle 20 umgelenkt und zu einer Walze 18 geführt, die bevorzugt einen Durchmesser von wenigstens 30 cm aufweist. Die Walze 18 ist Teil einer Ablenkvorrichtung 4.

Die Bahnware wird zwischen der Walze 18 und einem Walzenfräser 19 derart hindurchbewegt, dass eine erste Teilschicht 10 von der Ausgangsmembranbahnware entfernt wird und die gewünschte Spreitschicht 16 entsteht. Die Spreitschicht 16, die nun die Bahnware bildet, wird über eine weitere Ablenkrolle 21 geführt, bevor sie z.B. auf einer Rolle aufgewickelt und/oder weiterverarbeitet wird.

Die Walze 18 wird durch wenigstens eine Kaltluftdüse 28 in einem ersten Bereich (Kühlsektor 24) abgekühlt. In der bevorzugten Ausführungsform gemäß Figur 3, sind drei Kaltluftdüsen 28 vorgesehen. Die Kaltluftdüsen 28 sind derart angeordnet, dass im Zulaufbereich der Membran bis zu der Position eines als Walzenfräser 19 ausgebildeten Schneidwerkzeugs abgekühlt. Die Abkühlung geschieht auf einem Temperaturniveau unterhalb des Schmelzpunktes der verwendeten Tränkflüssigkeit, im Fall von Wasser also unterhalb des Gefrierpunktes, derart, dass die Ausgangsmembran 2 in dem Kühlsektor 24 an die Walze 18 anfriert und fixiert wird. Damit entspricht die Bewegungsgeschwindigkeit der Ausgangsmembran 2 der Walzengeschwindigkeit. Schubkräfte werden von der Ausgangsmembran 2 auf die Walze 18 übertragen.

Der Walzenfräser 19 weist bevorzugt eine Mehrzahl von kleinen Messern 19a auf, von denen aus Gründen der Übersichtlichkeit lediglich zwei in Figur 3 dargestellt sind. Mit Hilfe des Walzenfräsers 19 wird eine erste Teilschicht 10 von der Ausgangsmembran 2 abgetrennt, so dass die gewünschte Spreitschicht 16 als Bahnware verbleibt. Die bahnförmige Spreitschicht 16 wird mit der Walze 18 weiterbewegt zu einem Bereich (Heizsektor 25), in dem mehrere, beispielsweise zwei, Warmluftdüsen 29 angeordnet sind. Die zugeführte Wärmeenergie, die z.B. in Form von Warmluft oder Wärmestrahlung erfolgen kann, erwärmt die getränkte Spreitschicht 6 derart, dass die Tränkflüssigkeit schmilzt und sich verflüssigt, so dass die Spreitschicht 16 von der Walze 18 gelöst werden kann.

An der Walze 18 ist ein weiterer Abstreifer 23 vorgesehen, um überschüssige Tränkflüssigkeit in dem erwärmten Bereich (Heizsektor 25), in dem die Walzenoberfläche eine erhöhte, oberhalb des Schmelzpunktes der Tränkflüssigkeit liegende Temperatur hat, von der Walze 18 zu entfernen. Auf diese Weise werden überschüssige Reste entfernt, damit die sehr präzise und glatte Oberfläche der Walze 18 erhalten bleibt.

In Figur 3 ist zu erkennen, dass die Walze 18 vier Sektoren aufweist, den Kühlsektor 24, den Heizsektor 25, der eine erhöhte Oberflächentemperatur aufweist, und zwei neutrale Sektoren 26, 27. die zwischen dem Kühlsektor 24 und dem Heizsektor 25 angeordnet sind. In diesen Bereichen 26, 27 hat die Oberfläche der Walze 18 eine Temperatur, die unkontrolliert zwischen den Temperaturen in den jeweils benachbarten Sektoren 24 und 25 liegt.

Figur 4 zeigt ein Beispiel einer mit dem erfindungsgemäßen Verfahren hergestellten Spreitschicht 16 für ein optisches Analyseelement. Die Spreitschicht 16 ist zur Aufnahme einer zu analysierenden Körperflüssigkeit und zur gleichmäßigen Verteilung der Flüssigkeit über ihre Flüssigkeitsaustrittsseite 12 geeignet.

Die Spreitschicht 16 ist aus einer porösen Membran gebildet und entspricht der abgetrennten Teilschicht 11 der Membran 2. Die Spreitschicht 16 hat eine der Flüssigkeitsaustrittsseite 12 gegenüberliegende Flüssigkeitseintrittsseite 14, die aus der Oberseite 15 der Ausgangsmembran 2 gebildet wird. Die Dicke d ist mindestens 40 µm, bevorzugt mindestens 60 µm und besonders bevorzugt mindestens 80 µm und somit geringer als die Ausgangsdicke D der Membran 2.

Die Poren der Spreitschicht 16 stehen derart in Fluidverbindung miteinander, dass sie für eine Flüssigkeit durchlässig ist und eine an der Flüssigkeitseintrittsseite 14 eintretende Flüssigkeit zu der Flüssigkeitsaustrittsseite 12 transportiert und über die Flüssigkeitsaustrittsseite 12 verteilt. Die mittlere Porengröße der Spreitschicht 16 nimmt entlang ihrer Dicke d derart zu, dass die mittlere Porengröße an der Flüssigkeitseintrittsseite 14 größer ist als die an der Flüssigkeitsaustrittsseite 12. Bevorzugt ist die Zunahme der mittleren Porengröße stetig über die Dicke d, also über den Querschnitt der Spreitschicht 16. Die mittlere Porengröße an der Flüssigkeitsaustrittsseite 12 beträgt mindestens 1 µm, bevorzugt mindestens 2 µm und besonders bevorzugt mindestens 5 µm. Die mittlere Porengröße an der Flüssigkeitsaustrittsseite 12 ist höchstens 20 µm, bevorzugt höchstens 10 µm, besonders bevorzugt höchstens 7 µm.

Die Spreitschicht 16 hat an ihrer durch den Schneidprozess neu gebildeten Membranoberfläche, die die Flüssigkeitsaustrittsseite 12 ist, eine freie Porenfläche, die mindestens 70 % oder 75 %, bevorzugt mindestens 80 % oder 85 % und besonders bevorzugt mindestens 90 % oder 95 % beträgt. Damit ist die freie Porenfläche an der neu gebildeten Flüssigkeitsaustrittsseite 12 größer als die freie Porenfläche an der Unterseite 13 der Ausgangsmembran 2. Bevorzugt ist sie wenigstens zwei mal so groß, besonders bevorzugt wenigstens fünfmal so groß. Auch im Vergleich zu einer Unterseite einer Membran, die in einem herkömmlichen Herstellungsprozess hergestellt wird und die gleiche mittlere Porengröße hat, ist die freie Porenfläche der Flüssigkeitsaustrittsseite 12 größer, bevorzugt wenigstens 2 mal so groß. Somit unterscheidet sich die durch Schneiden hergestellte Spreitschicht 16 von einer Membran, die durch die bekannten Herstellungsprozesse wie beispielsweise Abschälen, Ätzen, Sintern oder durch Phaseninversion, entstehen.

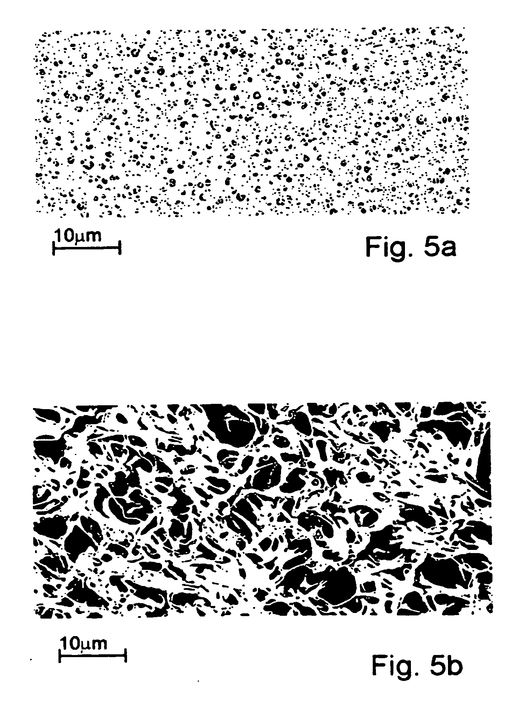

Fig. 5a zeigt ein rasterelektronenmikroskopisches Bild der Unterseite einer Ausgangsmembran BTS 65. Die freie Porenfläche, also die relative Offnungsfläche (Gesamtöffnungsfläche der Poren im Verhältnis zu der Gesamtfläche) ist nur gering und liegt unter 30 %.

Fig. 5b zeigt ein rasterelektronenmikroskopisches Bild der Membranoberfläche einer Spreitschicht, die durch spanabhebende Entfernung einer Teilschicht nach Tränkung mit PEG 1500, wie oben beschrieben, erhalten wurde. Die Vergrößerung des Bildes ist identisch mit der Vergrößerung des Bildes in Fig. 5a. Die freie Porenfläche ist deutlich vergrößert. Das Verhältnis aller Porenöffnungen zu der Gesamtfläche ist über 75 %.

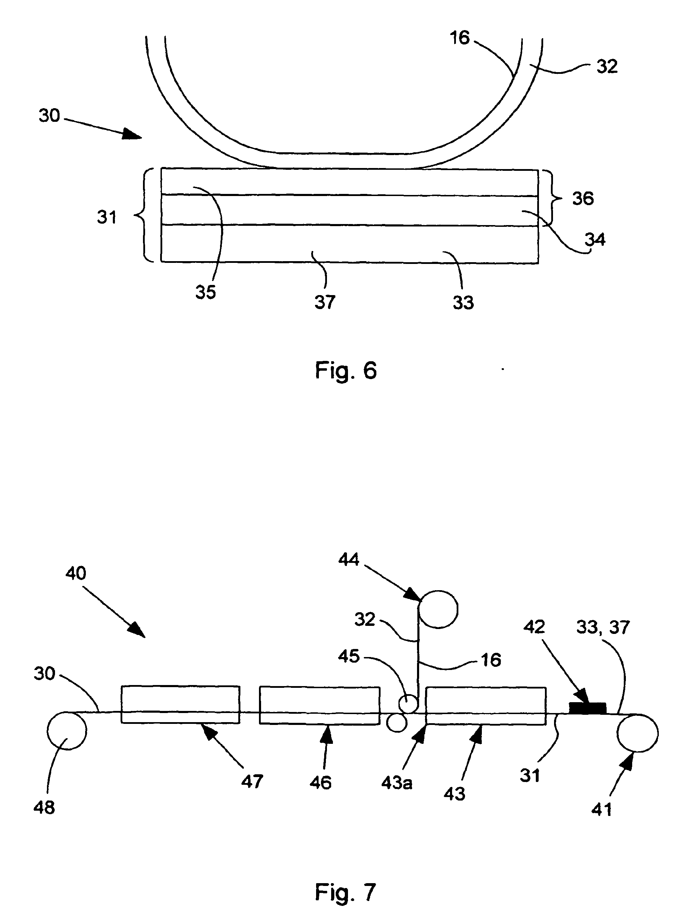

Fig. 6 zeigt ein Prinzipbild zur Herstellung eines erfindungsgemäßen Analyseelements 30, das ein Testfeld 31 und die erfindungsgemäße Spreitschicht 16, die in Form einer getrockneten, hydrophilen Membran 32 vorliegt, umfasst. Das Testfeld 31 weist eine Trägerfolie 33 auf, die die Tragschicht 37 des Analyseelements 30 bildet. Auf die transparente Trägerfolie 33 ist eine Reaktionsschicht 34 (Detektionsschicht) aufgebracht, die wenigstens ein Reagenz eines Reagenzsystems zum Nachweis eines Analyten enthält. Oberhalb der Reaktionsschicht 34 ist bei einem optischen Analyseelement 30 eine Reflexionsschicht 35 (z. B. aus Zinkoxid oder Titanoxid) angeordnet. Die Reaktionsschicht 34 und die Reflexionsschicht 35 bilden eine Testschicht 36.

Fig. 6 ist zu entnehmen, dass im Herstellungsprozess des Analyseelements 30 die Spreitschicht 16 (Membran 32) auf die angetrocknete aber noch feuchte Reflexionsschicht 35 der Testschicht 36 auflaminiert wird, also mit ihr in Kontakt gebracht wird. Bei einem Mehrschichtsystem wird die Membran 32 auf die obere (letzte) Schicht des Testfelds laminiert. Die Spreitschicht 16 wurde dabei durch Entfernen einer Teilschicht einer porösen Ausgangsmembran 2, wie sie vorstehend beschrieben ist, in einem vorgegebenen Abstand zur Oberseite 15 der Ausgangsmembran 2 gewonnen. Bevorzugt wurde die Spreitschicht 16 durch Schneiden parallel zur Oberseite 15 hergestellt.

Es hat sich bei Laboruntersuchungen als vorteilhaft erwiesen, die Membran 32 in Schlaufenform gebogen langsam auf die noch feuchte Testschicht 36 abzulegen, so dass sie auf die Testschicht 36 aufgerollt wird. Die Membran 32 saugt sich dabei an der feuchten Testschicht 36 an. Vorzugsweise wird ein Flüssigkeitsfilm auf der Oberseite des Testfelds 31 gebildet. Die vorhandene Flüssigkeit (Feuchte) übernimmt zunächst die Funktion eines Adhäsions-Klebers, der die Membran 32 an dem Testfeld 31 fixiert. Nach dem Trocknen ist ein fester und gut zu verarbeitender Verbund aus Testfeld 31 und Spreitschicht 16 (Membran 32) entstanden. In getrocknetem Zustand gewährleisten die polymeren Inhaltsstoffe des Testfelds, insbesondere der Reflexionsschicht 35 ein Anbinden der Membran 32.

Fig. 7 zeigt eine Produktionsanlage 40, bei der die Tragschicht 37 als bandförmige Trägerfolie 33 ausgebildet ist und auf einer Trägerrolle 41 aufgewickelt ist. Von dieser Trägerrolle 41 wird die Trägerfolie 33 abgewickelt und durchläuft eine Beschichtungseinheit 42, in der die Trägerfolie 33 mit der Testschicht 36 (Reaktionsschicht 34 und Reflexionsschicht 35) beschichtet wird. In einer ersten Trocknereinheit 43 wird das nun entstandene Testfeld 31 getrocknet, wobei es am Ende 43a der Trocknereinheit 43 noch die gewünschte Feuchte enthält.

Die efindungsgemäße Spreitschicht 16, die als bahnförmige Membran 32 auf einer Membranrolle 44 aufgewickelt ist, wird über eine Umlenkrolle 45 dem bandförmigen Testfeld 31 zugeführt. Dabei wird die Membran 32, wie anhand von Fig. 6 beschrieben, auf das Testfeld 31 aufgelegt und saugt sich an diesem fest. Hierdurch wird ein Auflaminieren der Membran verwirklicht.

Entscheidend in der Produktionsanlage 40 ist, dass die Testschicht 36 weder zu trocken noch zu nass ist. Vorteilhafterweise ist die Testschicht 36 derart feucht, wie es nach ca. 3 bis 11 Minuten Trockenzeit bei Raumtemperatur entspricht. Bevorzugt hat das Testelement 36 eine Feuchte zwischen 30 % und 50 %. Es hat sich als vorteilhaft erwiesen, wenn die relative Feuchte beim Auflaminieren zwischen 35 % und 47 % liegt. Besonders bevorzugt ist eine relative Feuchte von 40 % bis 45 %.

Nach dem Auflaminieren der Membran 32 auf das Testfeld 31 wird das entstandene Verbundelement, das das bandförmige Analyseelement 30 ist, in zwei weiteren Trockeneinheiten 46 und 47 getrocknet, bis das Analyseelementband auf einer Verbundrolle 48 aufgewickelt wird. Das Analyseelement 30 steht dann zur weiteren Verarbeitung zur Verfügung.

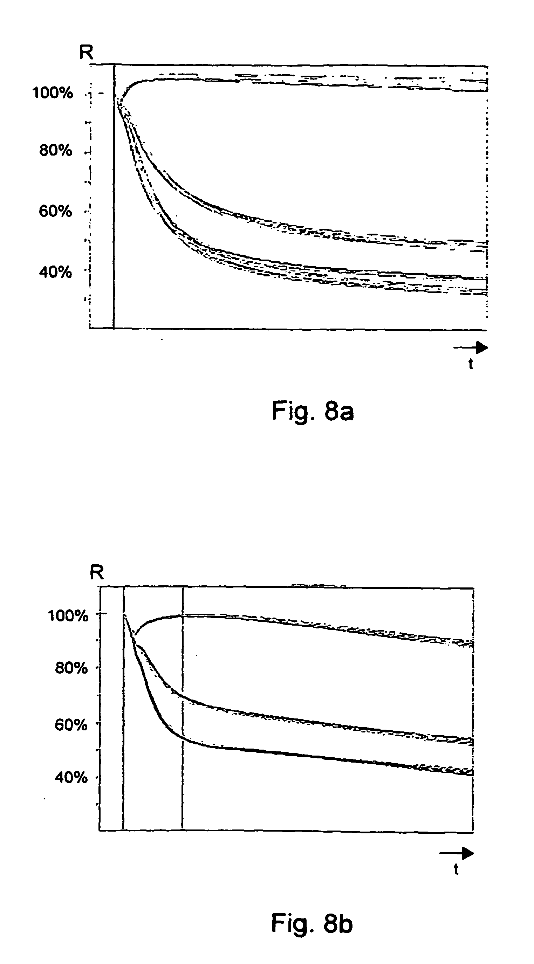

Die "Kinetikkurven" in den Figuren 8a, b zeigen den zeitlichen Verlauf einer Messung an unterschiedlichen Analyseelementen. Dabei wird die relative Remission R des empfangenen Lichts gemessen. Messgröße ist folglich das reflektierte Licht, das in Relation zu einer Leerwertmessung gesetzt wird, bei der keine Flüssigkeit die Reaktionsschicht 34 eines Analyseelements 30 benetzt. Die Figuren 8a, b zeigen jeweils drei Kurvenscharen von mehreren Messreihen mit Vollblut und unterschiedlichen Glucosegehalten. Die obere Kurvenschar (Messreihe) zeigt das Ergebnis von Vollblut mit einem Glucosegehalt von 0 mg/dl Glucose, während die Messungen der mittleren Kurvenschar mit einem Glucosegehalt von 250 mg/dl und die Messungen der unteren Kurvenschar mit einem Glucosewert von 545 mg/dl im Vollblut erfolgten.

In Fig. 8a sind die Messergebnisse mit einem herkömmlichen Analyseelement gezeigt, das ein monofiles Gewebe als Spreitschicht 16 verwendet. Das monofile Gewebe ist auf einem Testfeld aufgebracht. Fig. 8b zeigt die Messergebnisse bei der Verwendung eines erfindungsgemäßen Analyseelements 30 mit auflaminierter Membran 32. Der Vergleich der beiden Figuren 8a, 8b zeigt, dass bei Verwendung des erfindungsgemäßen Analyseelements 30 eine deutlich geringere Streuung innerhalb der jeweiligen Kurvenschar zu verzeichnen ist. Die Messergebnisse geben einen Aufschluss darüber, dass das Analyseelement 30 verbesserte Testeigenschaften aufweist und Messungen deutlich zuverlässiger und aussagekräftiger sind.

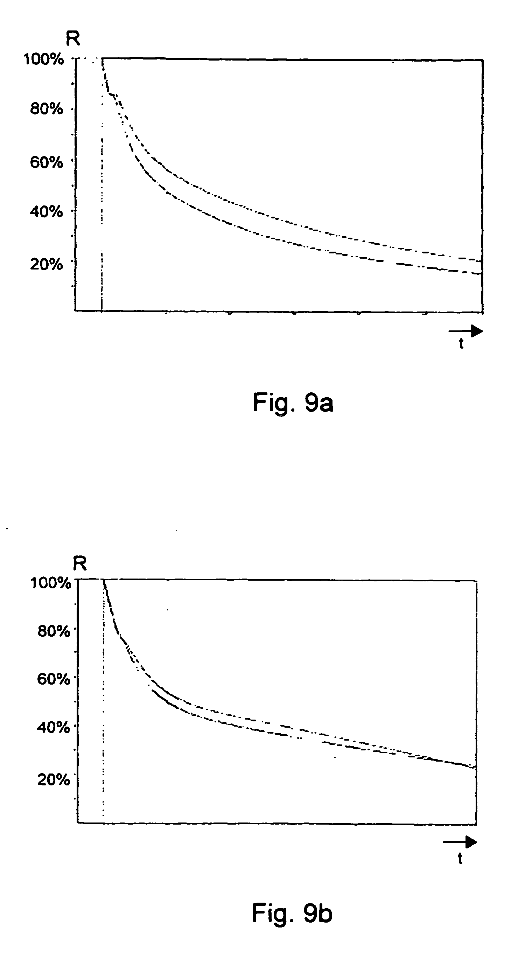

Die Figuren 9a und 9b zeigen jeweils wieder den Vergleich der beiden Analyseelemente (mit monofilem Gewebe und erfindungsgemäßer Spreitschicht 16). Es ist jeweils der Mittelwert der Remission R aus zwölf Messreihen dargestellt, wobei die Probenflüssigkeit aus Vollblut mit 250 mg/dl Glucoseanteil bestand. Der Hämatokritwert liegt bei 40 % (obere Kurve), 45 % (mittlere Kurve) und 50 % (untere Kurve). Der Vergleich der beiden Figuren 9a und 9b zeigt, dass bei Verwendung des erfindungsgemäßen Analyseelements 30 mit erfindungsgemäßer Spreitschicht 16 (Fig. 9b) ein deutlich geringerer Einfluss des Hämatokrits zu verzeichnen ist, da die drei Kurven deutlich näher beieinander liegen.

Zusammenfassend lässt sich festhalten, dass sich mit den erfindungsgemäßen Analyseelementen 30 im Vergleich zu Teststreifen oder Analyseelementen mit einem herkömmlichen Spreitnetz aus monofilem Gewebe eine deutlich verringerte Streuung der Messergebnisse und ein deutlich verringerter Einfluss des Hämatokrits auf die Ergebnisse abzeichnet. Es kommt folglich nicht zu den von der Fachwelt befürchteten Effekten, dass das Enzym oder der Indikator aus dem Reagenzsystem in die Membran 32 eingesaugt wird und der Signalhub verflachen würde und eine erschwerte Auswertung der Ergebnisse möglich sei. Im Gegenteil, es zeigt sich vielmehr, dass das erfindungsgemäße Analyseelement deutlich besser geeignet ist.

Darüber hinaus weist die Verwendung der erfindungsgemäßen Spreitschicht 16 in Form einer Membran 32, die auf das Testfeld 31 auflaminiert wird, bei der Verwendung von bandförmigen Analyseelementen 32 oder Teststreifen den Vorteil auf, dass auch bei kleinen Probenvolumina ein korrektes Ergebnis erzielt wird. Auch bei einer Zugspannung auf das bandförmige Analyseelement 30 ist durch den Prozess des Auflaminierens ein Abheben der Spreitschicht 16 von der Testschicht 36 des Testfelds 31 nicht gegeben, wie es bei der Verwendung von monofilen Spreitnetzen auftreten kann. Insofern lässt sich ein zuverlässiger Herstellungsprozess verwirklichen.

## Patentansprüche

1. Verfahren zur Herstellung einer Analyseelement-Spreitschicht, die dazu ausgebildet ist, in einem Analyseelements eine zu analysierende Flüssigkeit aufzunehmen und über eine Flüssigkeitsaustrittsseite der Spreitschicht gleichmäßig zu verteilen, wobei das Verfahren folgende Schritte umfasst:
Zuführen einer porösen Ausgangsmembran (2) mit einer Oberseite (15), einer Unterseite (13) und einer Ausgangsdicke D zwischen der Unterseite (13) und der Oberseite (15), wobei die Größe der Poren der Ausgangsmembran (2) über deren Ausgangsdicke D wenigstens abschnittsweise zunimmt,
Schneiden der Ausgangsmembran (2) in einem vorgegebenen Abstand parallel zu deren Oberseite (15) derart, dass eine Teilschicht der Ausgangsmembran entfernt wird, wobei
- die Spreitschicht (16) eine Teilschicht der Ausgangsmembran (2) ist, deren Dicke d geringer ist als die Ausgangsdicke D der Ausgangsmembran (2),
- die Poren der Spreitschicht (16) derartig miteinander in Fluidverbindung stehen, dass sie für eine Flüssigkeit durchlässig ist,
- die durch das Entfernen einer Teilschicht (11) der Ausgangsmembran (2) gebildete Membranoberfläche die Flüssigkeitsaustrittsseite (12) der Spreitschicht (16) ist und
- die mittlere Porengröße der Poren an der Flüssigkeitsaustrittsseite (12) der Spreitschicht (16) mindestens 1 µm beträgt.

2. Verfahren nach Anspruch 1, **dadurch gekennzeichnet, dass** nur an der Unterseite (13) der Ausgangsmembran (2) eine Teilschicht (10) entfernt wird, so dass die Oberseite (15) der Ausgangsmembran (2) auch eine Oberseite (15) der Spreitschicht (16) bildet, wobei die der Oberseite (15) gegenüberliegende Seite die Flüssigkeitsaustrittsseite (12) der Spreitschicht (16) ist.

3. Verfahren nach Anspruch 1 oder 2, **dadurch gekennzeichnet, dass** die Ausgangsmembran (2) auf einer Unterlage (6) befestigt wird und gemeinsam mit der Unterlage (6) als Materialbahn (7) zu einer Schneidvorrichtung (1) bewegt wird, mittels der die Ausgangsmembran (2) geschnitten wird.

4. Verfahren nach einem der vorhergehenden Ansprüche, **dadurch gekennzeichnet, dass** eine Schneidvorrichtung (1) eine Ablenkvorrichtung (4) einschließt, durch die eine die Ausgangsmembran (2) enthaltende Materialbahn (7) bei dem Produktionsprozess umgelenkt wird, wobei die Ausgangsmembran (2) beim Passieren der Ablenkvorrichtung (4) geschnitten wird.

5. Verfahren nach Anspruch 4, **dadurch gekennzeichnet, dass** die Ausgangsmembran (2) mit deren Oberseite (15) über die Ablenkvorrichtung (4) bewegt wird.

6. Verfahren nach einem der vorhergehenden Ansprüche, **dadurch gekennzeichnet, dass** die Ausgangsmembran (2) vor dem Entfernen oder während des Entfernens einer Teilschicht (10) mit einer Flüssigkeit getränkt wird, bevorzugt mit Wasser, einer wässrigen Lösung oder einem geschmolzenen wasserlöslichen Wachs.

7. Verfahren nach dem vorhergehenden Anspruch, **dadurch gekennzeichnet, dass** die Schneidvorrichtung (1) eine Walze umfasst und dass die mit Flüssigkeit getränkte Ausgangsmembran (2) an einem Abschnitt der Walze derart angefroren wird, dass die Ausgangsmembran (2) beim Schneiden an dem Abschnitt der Walze angefroren ist und mit der Walze bewegt wird.

8. Verfahren nach einem der vorhergehenden Ansprüche, **dadurch gekennzeichnet, dass** die Ausgangsmembran (2) eine stark asymmetrische Membran ist.

9. Verfahren nach einem der vorhergehenden Ansprüche, **dadurch gekennzeichnet, dass** die mittlere Porengröße an der Flüssigkeitsaustrittsseite (12) der Spreitschicht (16) mindestens 2 µm, bevorzugt mindestens 3 µm, besonders bevorzugt mindestens 5 µm und/oder höchstens 20 µm, bevorzugt höchstens 10 µm, besonders bevorzugt höchstens 7 µm beträgt.

10. Verfahren nach einem der vorhergehenden Ansprüche, **dadurch gekennzeichnet, dass** die mittlere Porengröße an der Oberseite (15) mindestens 10 µm, bevorzugt mindestens 20 µm, besonders bevorzugt mindestens 30 µm beträgt und/oder die mittlere Porengröße höchstens 60 µm, bevorzugt höchstens 50 µm, besonders bevorzugt höchstens 40 µm beträgt.

11. Verfahren nach einem der vorhergehenden Ansprüche, **dadurch gekennzeichnet, dass** die freie Porenfläche an der Flüssigkeitsaustrittsseite (12) der Spreitschicht (16) mindestens 70 %, bevorzugt mindestens 80 %, besonders bevorzugt mindestens 90 % beträgt.

12. Verfahren zur Herstellung eines Analyseelements zur Analyse einer Flüssigkeitsprobe, das ein Testelement und eine Analyseelement-Spreitschicht umfasst,
umfassend die folgenden Schritte:
- Zuführen einer porösen Ausgangsmembran (2) mit einer Oberseite (15), einer Unterseite (13) und einer Ausgangsdicke D zwischen der Unterseite (13) und der Oberseite (15), wobei die Größe der Poren der Ausgangsmembran (2) über deren Ausgangsdicke D wenigstens abschnittsweise zunimmt,
- Entfernen einer Teilschicht der Ausgangsmembran in einem vorgegebenen Abstand parallel zu deren Oberseite (15) derart, dass die Spreitschicht (16) eine Teilschicht (11) der Ausgangsmembran (2) mit einer Dicke d ist, die geringer ist als die Ausgangsdicke D der Ausgangsmembran (2), wobei
- die Poren der Spreitschicht (16) derartig miteinander in Fluidverbindung stehen, dass sie für eine Flüssigkeit durchlässig ist,
- die durch das Entfernen einer Teilschicht (10) der Ausgangsmembran (2) gebildete Membranoberfläche die Flüssigkeitsaustrittsseite (12) der Spreitschicht (16) ist und
- die mittlere Porengröße der Poren an der Flüssigkeitsaustrittsseite (12) der Spreitschicht (16) mindestens 1 µm beträgt,
- Zuführen der gebildeten Spreitschicht (16) zu einem Testfeld (31) mit einer Tragschicht (37) und einer Testschicht (36), wobei die Testschicht (36) eine Reaktionsschicht (34) mit wenigstens einem Reagenz umfasst, das Teil eines Reagenzsystems zum Nachweis eines Analyten in der Flüssigkeitsprobe ist,
- Aufbringen der Spreitschicht (16) auf das feuchte Testfeld (31) derart, dass die Flüssigkeitsaustrittsseite (12) der Spreitschicht (16) an einer Oberseite der Testschicht (36) anliegt, so dass die Spreitschicht (16) und das Testfeld (31) verbunden werden und gemeinsam das Analyseelement (30) bilden.

13. Verfahren nach einem der vorhergehenden Ansprüche, **dadurch gekennzeichnet, dass** die Spreitschicht (16) als hydrophile Membran vorliegt oder hydrophilisiert ist.

14. Spreitschicht eines Analyseelements zur Aufnahme einer zu analysierenden Flüssigkeit und zur gleichmäßigen Verteilung der Flüssigkeit über eine Flüssigkeitsaustrittsseite (12), insbesondere hergestellt nach einem Verfahren gemäß den vorhergehenden Ansprüchen, **dadurch gekennzeichnet, dass**
- die Spreitschicht (16) eine poröse Membran ist, die eine der Flüssigkeitsaustrittsseite (12) gegenüberliegende, eine Flüssigkeitseintrittsseite (14) bildende Oberseite hat;
- die Spreitschicht (16) eine Mehrzahl von Poren einschließt, die derart miteinander in Fluidverbindung stehen, dass die Spreitschicht (16) für eine Flüssigkeit durchlässig ist;
- die mittlere Porengrößen entlang der Dicke d der Spreitschicht (16) derart zunimmt, dass die mittlere Porengröße an der Flüssigkeitseintrittsseite (14) größer ist als die mittlere Porengrößen an der Ftüssigkeitsaustrittsseite (12);
- die mittlere Porengröße an der Flüssigkeitsaustrittsseite (12) mindestens 1 µm, bevorzugt mindestens 2 µm, besonders bevorzugt mindestens 5 µm beträgt und/oder die mittlere Porengröße der Flüssigkeitsaustrittsseite (12) höchstens 20 µm, bevorzugt höchstens 10 µm, besonders bevorzugt höchstens 7 µm beträgt, und
- die freie Porenfläche an der Flüssigkeitsaustrittsseite (12) der Spreitschicht (16) mindestens 70%, bevorzugt mindestens 80%, besonders bevorzugt mindestens 90% beträgt.

15. Analyseelement zur Analyse einer Flüssigkeitsprobe umfassend
- ein Testfeld (31) mit einer Tragschicht (37) und einer auf die Tragschicht (37) aufgebrachten Testschicht (36), wobei die Testschicht (36) eine Reaktionsschicht (34) mit wenigstens einem Reagenz aufweist und das Reagenz Teil eines Reagenzsystems zum Nachweis eines Analyten in einer Flüssigkeitsprobe ist, und
- eine Spreitschicht (16) zur Aufnahme der Flüssigkeitsprobe und zu dessen gleichmäßiger Verteilung über eine dem Testfeld (31) zugewandte Flüssigkeitsaustrittsseite (12),
**dadurch gekennzeichnet, dass**
- die Spreitschicht (16) eine poröse Membran ist, die eine der Flüssigkeitsaustrittsseite (12) gegenüberliegende, eine Flüssigkeitseintrittsseite (14) bildende Oberseite (15) hat;
- die Spreitschicht (16) eine Mehrzahl von Poren einschließt, die derart miteinander in Fluidverbindung stehen, dass die Spreitschicht (16) für eine Flüssigkeit durchlässig ist;
- die mittlere Porengröße entlang der Dicke der Spreitschichten (16) derart zunimmt, dass die mittlere Porengröße an der Flüssigkeitseintrittsseite (14) größer ist als die mittlere Porengröße an der Flüssigkeitsaustrittsseite (12);
- die mittlere Porengröße an der Flüssigkeitsaustrittsseite (12) mindestens 1 µm, bevorzugt mindestens 2 µm, besonders bevorzugt mindestens 5 µm beträgt und/oder die mittlere Porengröße der Flüssigkeitsaustrittsseite (12) höchstens 20 µm, bevorzugt höchstens 10 µm, besonders bevorzugt höchstens 7 µm beträgt; und
- die freie Porenfläche an der Flüssigkeitsaustrittsseite (12) der Spreitschicht (16) mindestens 70%, bevorzugt mindestens 80%. besonders bevorzugt mindestens 90% beträgt; und
- die Spreitschicht (16) mit dem Testfeld (31) derart in Kontakt steht, dass die Flüssigkeitsaustrittsseite (12) an der Testschicht (36) anliegt.

## Claims

1. Method for producing an analysis element spreading layer, which is implemented for absorbing a liquid to be analyzed in an analysis element and distributing it uniformly over a liquid outlet side of the spreading layer, the method comprising the following steps:
supplying a porous starting membrane (2) having a top side (15), a bottom side (13) and a starting thickness D between the bottom side (13) and the top side (15), the size of the pores of the starting membrane (2) increasing at least sectionally over its starting thickness D,
cutting the starting membrane (2) at a predefined distance parallel to its top side (15) in such a manner that a sublayer of the starting membrane is removed, wherein
- the spreading layer (16) is a sublayer of the starting membrane (2), whose thickness d is less than the starting thickness D of the starting membrane (2),
- the pores of the spreading layer (16) are a fluid connection to one another in such a manner that it is permeable to a liquid,
- the membrane surface formed by the removal of a sublayer (11) of the starting membrane (2) is the liquid outlet side (12) of the spreading layer (16), and
- the mean pore size of the pores on the liquid outlet side (12) of the spreading layer (16) is at least 1 µm.

2. Method according to claim 1, **characterized in that** a sublayer (10) is only removed on the bottom side (13) of the starting membrane (2), so that the top side (15) of the starting membrane (2) also forms a top side (15) of the spreading layer (16), the side opposite to the top side (15) being the liquid outlet side (12) of the spreading layer (16).

3. Method according to claim 1 or 2, **characterized in that** the starting membrane (2) is fastened on an underlay (6) and is moved jointly with the underlay (6) as a material web (7) to a cutting device (1), by means of which the starting membrane (2) is cut.

4. Method according to any one of the preceding claims, **characterized in that** a cutting device (1) comprises a deflection device (4), by which a material web (7) containing the starting membrane (2) is deflected during the production process, the starting membrane (2) being cut upon passing the deflection device (4).

5. Method according to claim 4, **characterized in that** the top side (15) of the starting membrane (2) is moved over the deflection device (4).

6. Method according to any one of the preceding claims, **characterized in that** the starting membrane (2), before the removal or during the removal of a sublayer (10), is impregnated with a liquid, preferably with water, an aqueous solution, or a molten water-soluble wax.

7. Method according to the preceding claim, **characterized in that** the cutting device (1) comprises a roller, and that the starting membrane (2) impregnated with liquid will be frozen on a section of the roller in such a manner that, during the cutting, the starting membrane (2) is frozen on the section of the roller and is moved with the roller.

8. Method according to any one of the preceding claims, **characterized in that** the starting membrane (2) is a strongly asymmetrical membrane.

9. Method according to any one of the preceding claims, **characterized in that** the mean pore size on the liquid outlet side (12) of the spreading layer (16) is at least 2 µm, preferably at least 3 µm, particularly preferably at least 5 µm and/or at most 20 µm, preferably at most 10 µm, particularly preferably at most 7 µm.

10. Method according to any one of the preceding claims, **characterized in that** the mean pore size on the top side (15) is at least 10 µm, preferably at least 20 µm, particularly preferably at least 30 µm and/or the mean pore size is at most 60 µm, preferably at most 50 µm, particularly preferably at most 40 µm.

11. Method according to any one of the preceding claims, **characterized in that** the free pore area on the liquid outlet side (12) of the spreading layer (16) is at least 70%, preferably at least 80%, particularly preferably at least 90%.

12. Method for producing an analysis element for analyzing a liquid sample, which comprises a test element and an analysis element spreading layer, comprising the following steps:
- supplying a porous starting membrane (2) having a top side (15), a bottom side (13), and a starting thickness D between the bottom side (13) and the top side (15), the size of the pores of the starting membrane (2) at least sectionally increasing over its starting thickness D,
- removing a sublayer of the starting membrane at a predefined distance parallel to its top side (15) in such a manner that the spreading layer (16) is a sublayer (11) of the starting membrane (2) having a thickness d, which is less than the starting thickness D of the starting membrane (2), wherein
- the pores of the spreading layer (16) are a fluid connection to one another in such a manner that they are permeable to a liquid,
- the membrane surface formed by the removal of a sublayer (10) of the starting membrane (2) is the liquid outlet side (12) of the spreading layer (16), and
- the mean pore size of the pores on the liquid outlet side (12) of the spreading layer (16) is at least 1 µm,
- supplying the spreading layer (16) formed to a test field (31) having a support layer (37) and a test layer (36), wherein the test layer (36) comprising a reaction layer (34) having at least one reagent, which is part of a reagent system for detecting an analyte in the liquid sample,
- applying the spreading layer (16) to the moist test field (31) in such a manner that the liquid outlet side (12) of the spreading layer (16) presses against a top side of the test layer (36), so that the spreading layer (16) and the test field (31) are connected and jointly form the analysis element (30).

13. Method according to any one of the preceding claims, **characterized in that** the spreading layer (16) is provided as a hydrophilic membrane or is hydrophilized.

14. Spreading layer of an analysis element for absorbing a liquid to be analyzed and for uniformly distributing the liquid over a liquid outlet side (12), in particular produced according to a method according to any one of the preceding claims, **characterized in that**
- the spreading layer (16) is a porous membrane, which has a top side opposite to the liquid outlet side (12), which forms a liquid inlet side (14);
- the spreading layer (16) comprises a plurality of pores, which are in fluid contact with one another in such a manner that the spreading layer (16) is permeable to a liquid;
- the mean pore size increases along the thickness d of the spreading layer (16) in such a manner that the mean pore size on the liquid inlet side (14) is greater than the mean pore size on the liquid outlet side (12);
- the mean pore size on the liquid outlet side (12) is at least 1 µm, preferably at least 2 µm, particularly preferably at least 5 µm and/or the mean pore size of the liquid outlet side (12) is at most 20 µm, preferably at most 10 µm, particularly preferably at most 7 µm, and
- the free pore area on the liquid outlet side (12) of the spreading layer (16) is at least 70%, preferably at least 80%, particularly preferably at least 90%.

15. Analysis element for analyzing a liquid sample comprising
- a test field (31) having a support layer (37) and a test layer (36) applied to the support layer (37), the test layer (36) having a reaction layer (34) with at least one reagent and the reagent being part of a reagent system for detecting an analyte in a liquid sample, and
- a spreading layer (16) for absorbing a liquid sample and for uniformly distributing it over a liquid outlet side (12) facing toward the test field (31),
**characterized in that**
- the spreading layer (16) is a porous membrane, which has a top side (15) opposite to the liquid outlet side (12), which forms a liquid inlet side (14);
- the spreading layer (16) comprises a plurality of pores, which are a fluid connection to one another in such a manner that the spreading layer (16) is permeable to a liquid;
- the mean pore size increases along the thickness of the spreading layers (16) in such a manner that the mean pore size on the liquid inlet side (14) is greater than the mean pore size on the liquid outlet side (12);
- the mean pore size on the liquid outlet side (12) is at least 1 µm, preferably at least 2 µm, particularly preferably at least 5 µm and/or the mean pore size of the liquid outlet side (12) is at most 20 µm, preferably at most 10 µm, particularly preferably at most 7 µm; and
- the free pore area on the liquid outlet side (12) of the spreading layer (16) is at least 70%, preferably at least 80%, particularly preferably at least 90%; and
- the spreading layer (16) is in contact with the test field (31) in such a manner that the liquid outlet side (12) presses against the test layer (36).

## Revendications

1. Procédé de fabrication d'une couche d'étalement d'un élément d'analyse, qui est conçue pour recevoir dans un élément d'analyse un liquide à analyser et pour diffuser celui-ci uniformément à travers un côté sortie de liquide de la couche d'étalement, le procédé comprenant les étapes suivantes :
amener une membrane de départ poreuse (2) ayant un côté supérieur (15), un côté inférieur (13) et une épaisseur initiale D entre le côté inférieur (13) et le côté supérieur (15), la taille des pores de la membrane de départ (2) augmentant au moins par sections sur son épaisseur initiale D,
couper la membrane de départ (2) à une distance prédéfinie parallèlement à son côté supérieur (15) de sorte à enlever une couche partielle de la membrane de départ,
- la couche d'étalement (16) étant une couche partielle de la membrane de départ (2), dont l'épaisseur d est inférieure à l'épaisseur initiale D de la membrane de départ (2),
- les pores de la couche d'étalement (16) étant en communication fluidique les uns avec les autres de sorte qu'ils sont perméables à un liquide,
- la surface de membrane formée par l'enlèvement d'une couche partielle (11) de la membrane de départ (2) étant le côté sortie de liquide (12) de la couche d'étalement (16) et
- la taille moyenne des pores sur le côté sortie de liquide (12) de la couche d'étalement (16) étant au moins de 1 µm.

2. Procédé selon la revendication 1, **caractérisé en ce qu'**une couche partielle (10) est enlevée seulement sur le côté inférieur (13) de la membrane de départ (2) de sorte que le côté supérieur (15) de la membrane de départ (2) forme aussi un côté supérieur (15) de la couche d'étalement (16), le côté faisant face au côté supérieur (15) étant le côté sortie de liquide (12) de la couche d'étalement (16).

3. Procédé selon la revendication 1 ou 2, **caractérisé en ce que** la membrane de départ (2) est fixée sur un support (6) et se déplace ensemble avec le support (6), comme bande de matériau (7), vers un dispositif de coupe (1) au moyen duquel la membrane de départ (2) est coupée.

4. Procédé selon l'une des revendications précédentes, **caractérisé en ce qu'**un dispositif de coupe (1) inclut un dispositif de déviation (4) au moyen duquel une bande de matériau (7) contenant la membrane de départ (2) est déviée durant le processus de production, la membrane de départ (2) étant coupée lorsqu'elle passe sur le dispositif de déviation (4).

5. Procédé selon la revendication 4, **caractérisé en ce que** la membrane de départ (2) se déplace avec son côté supérieur (15) sur le dispositif de déviation (4).

6. Procédé selon l'une des revendications précédentes, **caractérisé en ce que** la membrane de départ (2), avant ou pendant l'enlèvement d'une couche partielle (10), est imbibée d'un liquide, de préférence d'eau, d'une solution aqueuse ou d'une cire fondue soluble dans l'eau.

7. Procédé selon l'une des revendications précédentes, **caractérisé en ce que** le dispositif de coupe (1) comprend un cylindre et **en ce que** la membrane de départ (2) imbibée de liquide est attachée par le gel sur une partie du cylindre de sorte que la membrane de départ (2), au moment de couper, se trouve attachée par le gel sur cette partie du cylindre et se déplace avec le cylindre.

8. Procédé selon l'une des revendications précédentes, **caractérisé en ce que** la membrane de départ (2) est une membrane fortement asymétrique.

9. Procédé selon l'une des revendications précédentes, **caractérisé en ce que** la taille moyenne des pores sur le côté sortie de liquide (12) de la couche d'étalement (16) est au moins de 2 µm, de préférence au moins de 3 µm, de manière particulièrement préférée au moins de 5 µm et/ou au plus de 20 µm, de préférence au plus de 10 µm, de manière particulièrement préférée au plus de 7 µm.

10. Procédé selon l'une des revendications précédentes, **caractérisé en ce que** la taille moyenne des pores sur le côté supérieur (15) est au moins de 10 µm, de préférence au moins de 20 µm, de manière particulièrement préférée au moins de 30 µm et/ou ladite taille moyenne des pores est au plus de 60 µm, de préférence au plus de 50 µm, de manière particulièrement préférée au plus de 40 µm.

11. Procédé selon l'une des revendications précédentes, **caractérisé en ce que** la surface poreuse libre sur le côté sortie de liquide (12) de la couche d'étalement (16) est au moins de 70 %, de préférence au moins de 80 %, de manière particulièrement préférée au moins de 90 %.

12. Procédé de fabrication d'un élément d'analyse destiné à analyser un échantillon de liquide, comprenant un élément de test et une couche d'étalement d'élément d'analyse,
comprenant les étapes suivantes :
- amener une membrane de départ poreuse (2) ayant un côté supérieur (15), un côté inférieur (13) et une épaisseur initiale D entre le côté inférieur (13) et le côté supérieur (15), la taille des pores de la membrane de départ (2) augmentant au moins par sections sur son épaisseur initiale D,
- enlever une couche partielle de la membrane de départ à une distance prédéfinie parallèlement à son côté supérieur (15) de sorte que la couche d'étalement (16) est une couche partielle (11) de la membrane de départ (2) avec une épaisseur d qui est inférieure à l'épaisseur initiale D de la membrane de départ (2),
- les pores de la couche d'étalement (16) étant en communication fluidique les uns avec les autres de sorte qu'ils sont perméables à un liquide,
- la surface de membrane formée par l'enlèvement d'une couche partielle (10) de la membrane de départ (2) étant le côté sortie de liquide (12) de la couche d'étalement (16) et
- la taille moyenne des pores sur le côté sortie de liquide (12) de la couche d'étalement (16) étant au moins de 1 µm,
- amener la couche d'étalement formée (16) vers une zone de test (31) présentant une couche de support (37) et une couche de test (36), la couche de test (36) comprenant une couche réactive (34) avec au moins un réactif qui fait partie d'un système de réactif destiné à détecter un analyte dans l'échantillon de liquide,
- déposer la couche d'étalement (16) sur la zone de test (31) humide de façon à ce que le côté sortie de liquide (12) de la couche d'étalement (16) soit appliqué contre un côté supérieur de la couche de test (36), permettant ainsi d'assembler la couche d'étalement (16) et la zone de test (31) pour former ensemble l'élément d'analyse (30).

13. Procédé selon l'une des revendications précédentes, **caractérisé en ce que** la couche d'étalement (16) se présente sous forme de membrane hydrophile ou est hydrophilisée.

14. Couche d'étalement d'un élément d'analyse destinée à recevoir un liquide à analyser et à diffuser le liquide uniformément à travers un côté sortie de liquide (12), en particulier fabriquée conformément à un procédé selon les revendications précédentes, **caractérisée en ce que**
- la couche d'étalement (16) est une membrane poreuse qui a un côté supérieur faisant face au côté sortie de liquide (12) et formant un côté entrée de liquide (14) ;
- la couche d'étalement (16) comprend une pluralité de pores qui sont en communication fluidique les uns avec les autres de sorte que la couche d'étalement (16) est perméable à un liquide ;
- la taille moyenne des pores augmente le long de l'épaisseur d de la couche d'étalement (16) de sorte que la taille moyenne des pores sur le côté entrée de liquide (14) est plus grande que la taille moyenne des pores sur le côté sortie de liquide (12) ;
- la taille moyenne des pores sur le côté sortie de liquide (12) est au moins de 1 µm, de préférence au moins de 2 µm, de manière particulièrement préférée au moins de 5 µm et/ou la taille moyenne des pores du côté sortie de liquide (12) est au plus de 20 µm, de préférence au plus de 10 µm, de manière particulièrement préférée au plus de 7 µm, et
- la surface poreuse libre sur le côté sortie de liquide (12) de la couche d'étalement (16) est au moins de 70 %, de préférence au moins de 80 %, de manière particulièrement préférée au moins de 90 %.

15. Élément d'analyse destiné à analyser un échantillon de liquide, comprenant
- une zone de test (31) présentant une couche de support (37) et une couche de test (36) déposée sur la couche de support (37), la couche de test (36) comprenant une couche réactive (34) avec au moins un réactif et le réactif faisant partie d'un système de réactif destiné à détecter un analyte dans un échantillon de liquide, et
- une couche d'étalement (16) destinée à recevoir l'échantillon de liquide et à diffuser celui-ci uniformément à travers un côté sortie de liquide (12) faisant face à la zone de test (31),
**caractérisé en ce que**
- la couche d'étalement (16) est une membrane poreuse qui a un côté supérieur (15) faisant face au côté sortie de liquide (12) et formant un côté entrée de liquide (14) ;
- la couche d'étalement (16) comprend une pluralité de pores qui sont en communication fluidique les uns avec les autres de sorte que la couche d'étalement (16) est perméable à un liquide.
- la taille moyenne des pores augmente le long de l'épaisseur des couches d'étalement (16) de sorte que la taille moyenne des pores sur le côté entrée de liquide (14) est plus grande que la taille moyenne des pores sur le côté sortie de liquide (12) ;
- la taille moyenne des pores sur le côté sortie de liquide (12) est au moins de 1 µm, de préférence au moins de 2 µm, de manière particulièrement préférée au moins de 5 µm et/ou la taille moyenne des pores du côté sortie de liquide (12) est au plus de 20 µm, de préférence au plus de 10 µm, de manière particulièrement préférée au plus dé 7 µm, et
- la surface poreuse libre sur le côté sortie de liquide (12) de la couche d'étalement (16) est au moins de 70 %, de préférence au moins de 80 %, de manière particulièrement préférée au moins de 90 % ; et
- la couche d'étalement (16) est en contact avec la zone de test (31) de sorte que le côté sortie de liquide (12) est appliqué contre la couche de test (36).
